# EUROPEAN PATENT SPECIFICATION

(11) **EP 0 997 137 B1**
(45) Date of publication and mention of the grant of the patent: **05.06.2002**
(21) Application number: 99120487.6
(22) Date of filing: 15.10.1999
(51) Int. Cl.: A61K 7/48, A61K 7/06, A61K 31/12, A61K 31/36, A61K 7/50

(54) **Use of piperonal as an anti-inflammatory additive to cosmetics and medicaments**
Verwendung von Piperonal als entzündungshemmender Zusatz für kosmetische Präparate und Arzneimittel
Utilisation du pipéronal comme additif anti-inflammatoire pour compositions cosmétiques et médicaments

(30) Priority: 15.10.1998 DE 19847691
(43) Date of publication of application: 03.05.2000
(73) Proprietor: Société des Produits Nestlé S.A., 1800 Vevey (CH)
(72) Inventor: Stiehm, Thomas, 33739 Bielefeld (DE); Baur, Markus, 1066 Epalinges (CH)

(56) References cited:
- WO-A-90/09738
- S. PEOCK: "Arena Test with Piperonal, A New Louse Repellent" JOURNAL OF THE ROYAL SOCIETY OF HEALTH, vol. 113, no. 6, 1993, pages 292-294, XP000869531
- R. J. ANTO ET AL.: "Anti-inflammatory Activity of Natural and Synthetic Curcuminoids" PHARMACY AND PHARMACOLOGY COMMUNICATIONS, vol. 4, no. 2, 1998, page 103-106 XP000866535

## Description

The present invention relates to uses of piperonal, which arise from the recent realization that piperonal has anti-inflammatory properties.

Piperonal (heliotropin, piperonylaldehyde, 3,4-(methylenedioxy)benzaldehyde) is a known compound which occurs in various flower oils and types of vanilla, can be prepared synthetically and, because of its sweetish, heliotropic-like odour, is used in perfumery, in particular for the perfuming of soap. Piperonal is also used as a repellent against head lice (J R Soc Health. 1993; 113(6): 292-294). The document Pharmacy and Pharmacology Communications, vol. 4 no. 2, 1998, 103-106 discloses the use of piperonal curcuminoid as anti-inflammatory agent.

In connection with the described known applications of piperonal in cosmetics, various investigations have already been carried out which aimed to confirm the toxicological acceptability of piperonal for the known applications. However, useful medicament effects of piperonal have hitherto not been disclosed.

The present invention is based on the realization, made on the basis of in vitro experiments using a recognized test model, that piperonal has an anti-inflammatory effect. This realization makes piperonal interesting for a large number of new types of applications for which it has hitherto not been used or has been used in a different form and for which it would also not have been used without the above new realization, e.g. also because in the case of such applications perfuming is not usual or would appear inappropriate, or other perfume additives were common.

The present invention thus relates to the use of piperonal (heliotropin) as an anti-inflammatory additive in the preparation of cosmetic products and medicaments in quite general terms.

An addition of an anti-inflammatory agent, which is additionally notable for a pleasant odour, is suitable in particular for cosmetic and medicinal products for external applcation to the skin, it being possible to mention as examples of such products, in addition to soaps and shampoos, ointments, solutions, cleansing wipes, sprays and eye drops, in each case in the form of products for cosmetic applications, but also in the form of products for medicinal purposes. A particularly interesting aspect also appears to be an addition of piperonal to cosmetic and medicinal products of the emulsion type (W/O and O/W emulsions) such as creams and lotions. In addition, piperonal can also be added to shampoos to improve their skin compatibility, in particular mild shampoos for babies and small children.

Other products which come into contact with the skin which may be mentioned are plasters, in which piperonal can be incorporated into the adhesive.

The anti-inflammatory effect of an addition of piperonal can, however, also be utilized in typical medicaments, where piperonal can be used in addition to other active ingredients which are decisive for the type of medicament and determine its application. Piperonal can, however, also be used as the sole active ingredient in medicaments whose aim is to inhibit inflammation. Examples which may be mentioned are medicaments for treating inflammation due, for example, to sunburn or nappy rash, for wound healing and for the treatment of chronic inflammatory diseases such as psoriasis and constitutional eczema.

As is the case for cosmetics, medicament forms which are applied externally (topically) are again of primary interest, for example medicaments in the form of emulsions for dermatological purposes, although medicaments which act transdermally should also be included, in which the medicament active ingredients are absorbed via the skin, including active ingredient plasters.

An anti-inflammatory piperonal additive is, however, also suitable for medicaments which are applied internally, where the medicament active ingredients are absorbed via mucous membranes, for example in the case of medicaments administered rectally or vaginally in suppository form, or else in the case of medicaments administered perorally, in which piperonal can develop a protective or healing anti-inflammatory action in the digestive tract.

For the use of piperonal as anti-inflammatory additive for cosmetics and medicaments, there are no critical limitations with regard to the amounts applied, the lower limit of the amounts which can be used being predetermined by the amount from which a significant anti-inflammatory action can be sensibly assumed. The upper limit of the amounts applied can be predetermined in the individual case on the basis of various types of considerations, which include: undesirably high odour or taste effects, stability considerations, compatibility with the carrier medium, such as, for example, an ointment base or a liquid composition, or other active ingredients, solubility factors and transportation factors and more besides.

In general, the amount applied is in a broad range from 0.01 to 2.0% by weight, based on the cosmetic product or medicament, the amounts applied usually being in the range from 0.01 to 1.0% by weight and preferably in the range from 0.01 to 0.5% by weight.

### Experimental part - Examples

The anti-inflammatory properties were established in the experiments described in more detail below:

### In vitro experiments

The anti-inflammatory properties of piperonal were demonstrated in an in vitro assay model using immortalized skin cells (keratinocytes). These keratinocytes (cf. WO97/23602) are treated in this connection with inflammation-promoting substances, more specifically with a known skin irritant in the form of a phorbol ester (12-O-tetradecanoylphorbol acetate, abbreviated to TPA), and as a response to this treatment they display a typical inflammatory metabolic reaction, in particular an increase in the secretion of the inflammation-promoting cytokine TNF-α (tumour necrosis factor alpha).

A substance which inhibits the secretion of TNF-α in the case of TPA-treated keratinocytes in the above model in a dose-dependent manner is regarded as a potentially anti-inflammatory substance.

Specifically, the test is carried out by subjecting in vitro human keratinocytes, immortalized in culture, to an inflammation-promoting treatment using the abovementioned 12-O-tetradecanoylphorbol 13-acetate (TPA). The immortalized keratinocyte cell lines used are the cell lines DK2, FK2 or else DK7, which are described in the published International Patent Application WO 97/23602. The cell lines FK2 and DK2 were deposited on 5 October 1995 in accordance with the Budapest Treaty at the DSM depository (Deutsche Sammlung für Mikroorganismen und Zellkulturen GmbH, Mascheroder Weg 1b, D-38124 Braunschweig, Germany) and were given the deposit names DSM ACC2240 and DSM ACC2238. The cell line DK7-NR was deposited on 19 March 1998 at the Pasteur Institute, Paris (CNCM) under the number

I-1996. It is also possible to use an immortalized keratinocyte line HaCaT, which is described by Boukamp et al. in J. Cell. Biol., 106, 761-771, 1988).

### IN VITRO TEST PROCEDURE

Petri dishes (6 cm diameter), which contain 4 ml of the serum-free nutrient medium NR-2 (cf. WO97/23602; Biofluids), were inoculated with the abovementioned immortalized keratinocyte cell lines. As soon as confluence of the cell lawn was observed, the nutrient medium was replaced by a fresh, calcium-enriched nutrient medium NR-2 (1.5 mM CaCl₂). The dishes were incubated for 2 days, the nutrient medium was replaced by fresh nutrient medium and the dishes were incubated for a further 2 days. The cells were then nourished for 2 days with a calcium-enriched nutrient medium NR-2 (CaCl₂, 1.5 mM) which contained piperonal in the desired test concentration. In parallel, an identical batch without piperonal additive was prepared.

During the last 24 h of the above mentioned incubation, TPA in ethanol or a corresponding amount of ethanol was added in an amount of 10 ng/ml. The supernatants were then isolated and, by means of a known ELISA assay (Ruwag AG, Zurich, Switzerland) using specific antibodies to TNF-α (BioSource Inter., US, catalogue numbers KHC0012 and KHC3013), the concentration of TNF-α in the supernatant was determined.

### RESULTS

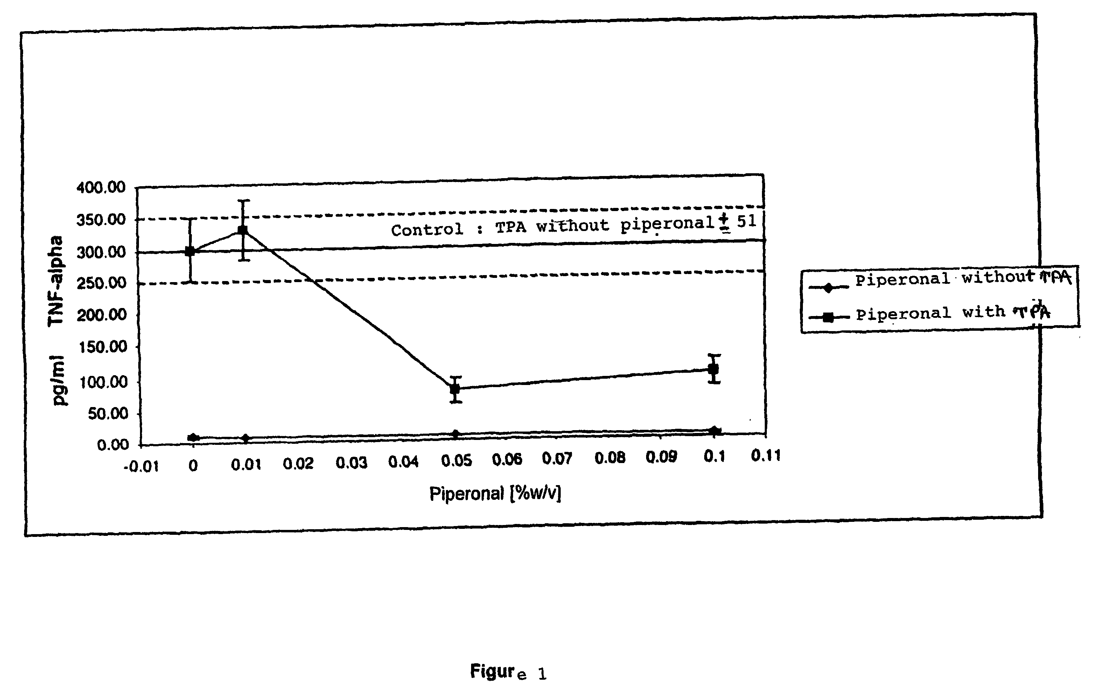

The results obtained are summarized in Table 1 below and shown graphically in Fig. 1.

Figure 1 shows, in graph form, the effect of varying concentrations of piperonal on the secretion of TNF-α by TPA-treated human keratinocytes.

**Table 1**

| Treatment | Amount of TNF-α determined (pg/ml) | ± SD | % of the control | % inhibition |
|---|---|---|---|---|
| Control: TPA without piperonal | 299.5 | 51.2 | 100.0 | 0 |
| TPA + 0.01% of piperonal | 331.2 | 47.1 | 110.6 | none |
| TPA + 0.05% of piperonal | 78.1 | 19.2 | 26.1 | 73.9 |
| TPA + 0.1% of piperonal | 100.6 | 19.8 | 33.6 | 66.4 |
| TPA + hydrocortisone | 220.4 | 43.5 | 73.6 | 26.4 |
| Control TPA: no TPA | 11.0 | 3.1 | - | - |
| No TPA + 0.01% of piperonal | 7.9 | 2.1 | - | - |
| No TPA + 0.05% of piperonal | 8.9 | 1.1 | - | - |
| No TPA + 0.1% of piperonal | 6.3 | 2.5 | - | - |
| Explanations: | | | | |
| TPA = 12-O-tetradecanoylphorbol 13-acetate | | | | |
| SD = percentage standard deviation | | | | |
| - = not applicable | | | | |

The results clearly show that piperonal has a strong inhibiting effect on the TNF-α secretion of keratinocytes which are treated with TPA, this effect being significantly stronger than that for the known anti-inflammatory hydrocortisone.

At the same time, the control experiments without TPA show that piperonal itself - within the limits of measurement accuracy - does not cause an increase in TNF-α secretion, i.e. is itself not to be regarded as triggering inflammation.

In addition, other experiments have established that piperonal does not exhibit cytotoxic activity during the treatment of the abovementioned keratinocytes in the concentrations used in the experiments.

The proven suppression of the secretion of the inflammation-triggering substance TNF-α in the abovementioned test by piperonal thus suggests a strong, hitherto unknown anti-inflammatory action of piperonal.

### In vivo test

The test investigated whether a gel containing piperonal influences an erythema produced by UV irradiation.

In the in vivo test described, the severity of the erythema produced by UV irradiation was determined by measuring the capillary skin perfusion in the irradiated region and by evaluating the redness using a redness index. The action of the piperonal-containing gel was tested in this case in comparison with untreated skin.

In the untreated case, the skin perfusion increased to 3.5 times the normal value 6 h after the erythema had been triggered by local UV irradiation of an area of human skin, and the increased value remained essentially unchanged for 24 h and fell to 2.3 times the normal value after 48 h.

If the skin was treated immediately after irradiation with a gel containing 0.2% by weight of piperonal (Carbopol gel), the skin perfusion increased only to 2.3 times the normal value after irradiation identical to that above and dropped to 1.7 times the normal value after 24 h. After 48 h a value was achieved which was 1.3 times the normal value.

The piperonal-containing gel also achieved a significant reduction in skin redness, specifically a reduction to half that of the untreated erythema after 6 h, 24 h and 48 h.

Some more illustrative formulations for cosmetic products containing a piperonal additive according to the invention are listed below. All of the amounts are given as percentage amounts by weight.

### Example 1 - gel

| | |
|---|---|
| Carbomer | 0.7% |
| Sodium hydroxide, 10% strength | 0.7% |
| Piperonal | 0.2% |
| Preservative | q.s. |
| Water | Remainder to 100% |

### Example 2 - Body lotion

| | |
|---|---|
| Polyglyceryl-3 diisostearate | 2.0% |
| Mineral oil | 8.0% |
| Isopropyl palmitate | 5.0% |
| Octyldodecanol | 4.0% |
| Carbomer | 0.3% |
| Sodium cocoylglutamate | 0.2% |
| Sodium hydroxide, 10% strength | 1.2% |
| Preservative | q.s. |
| Perfume | q.s. |
| Piperonal | 0.2% |
| Water | Remainder to 100% |

### Example 3 - mild shampoo

| | |
|---|---|
| Sodium lauryl sulphate | 7.0% |
| Cocamidopropylbetain | 2.0% |
| Sodium lauryl sulphonosuccinate | 2.0% |
| Piperonal | 0.2% |
| Sodium chloride | q.s. |
| Preservative | q.s. |
| Perfume | q.s. |
| Water | Remainder to 100% |

## Claims

1. Use of piperonal as an anti-inflammatory additive in the preparation of cosmetic products and medicaments.

2. Use of piperonal according to Claim 1 as an anti-inflammatory additive to cosmetic and medicinal products for skincare and haircare.

3. Use of piperonal according to Claim 1 as an anti-inflammatory additive to cosmetics or medicaments which are applied topically or are transdermal, in the form of shampoos, W/O and O/W emulsions, ointments, solutions, cleansing wipes, sprays and eye drops.

4. Use of piperonal according to Claim 1 as an anti-inflammatory additive to medicaments which are administered perorally, rectally or vaginally.

## Patentansprüche

1. Verwendung von Piperonal (Heliotropin) als entzündungshemmender Zusatz bei der Herstellung von kosmetischen Produkten und Arzneimitteln.

2. Verwendung von Piperonal nach Anspruch 1 als entzündungshemmender Zusatz zu kosmetischen und medizinischen Produkten für die Hautpflege und die Haarpflege.

3. Verwendung von Piperonal nach Anspruch 1 als entzündungshemmender Zusatz zu Kosmetika oder topisch anzuwendenden oder transdermalen Arzneimitteln in Form von Shampoos, W/O und O/W Emulsionen, Salben, Lösungen, Reinigungstüchern, Sprays und Augentropfen.

4. Verwendung von Piperonal nach Anspruch 1 als entzündungshemmender Zusatz zu peroral, rektal oder vaginal anzuwendenden Arzneimitteln.

## Revendications

1. Utilisation de pipéronal comme additif anti-inflammatoire dans la préparation de produits cosmétiques et de médicaments.

2. Utilisation de pipéronal suivant la revendication 1 comme additif anti-inflammatoire pour des produits cosmétiques et des produits médicamenteux pour les soins cutanés et les soins capillaires.

3. Utilisation de pipéronal suivant la revendication 1 comme additif anti-inflammatoire- pour des produits cosmétiques ou des médicaments qui sont appliqués localement ou qui sont des agents transdermiques, sous forme de shampooings, d'émulsions eau/huile et huile/eau, de pommades, de solutions, de lingettes nettoyantes, de liquides d'atomisation et de gouttes ophtalmiques.

4. Utilisation de pipéronal suivant la revendication 1 comme additif anti-inflammatoire pour des médicaments qui sont administrés par voie perorale, rectale ou vaginale.
